Europäisches Patentamt

European Patent Office

Office européen des brevets

(11) Numéro de publication: **0 273 836**
**A2**

## (12) DEMANDE DE BREVET EUROPEEN

(21) Numéro de dépôt: **87420321.9**

(22) Date de dépôt: **27.11.87**

(51) Int. Cl.⁴: **C 07 D 251/34**
**C 08 G 18/78**

(30) Priorité: **02.12.86 FR 8617012**

(43) Date de publication de la demande:
**06.07.88 Bulletin 88/27**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Robin, Jean**
**21, rue Duguesclin**
**F-69006 Lyon (FR)**

**Blind, André**
**10, rue Martin Basse**
**F-69300 Caluire et Cuire (FR)**

(74) Mandataire: **Vignally, Noel et al**
**RHONE-POULENC INTERSERVICES Service Brevets**
**Chimie Centre de Recherches de Saint-Fons B.P. 62**
**F-69192 Saint-Fons Cedex (FR)**

(54) Polyisocyanates à groupement isocyanurique purs et procédé d'obtention de ces polyisocyanates.

(57) La présente invention concerne des polyisocyanates à groupement isocyanurique contenant une quantité de diisocyanate inférieure ou égale à 0,03 % en poids et une quantité de dimère inférieure ou égale à 1,0 % en poids.

La présente invention concerne également l'isolement et la purification des polyisocyanates à groupement isocyanurate, et plus particulièrement de ces polyisocyanates préparés par cyclotrimérisation catalytique d'un diisocyanate aliphatique, cycloaliphatique ou arylaliphatique.

Elle consiste plus particulièrement à traiter le polyisocyanate à groupement isocyanurique brut par un gaz inerte à l'état liquide ou supercritique.

EP 0 273 836 A2

**Description**

POLYISOCYANATES A GROUPEMENT ISOCYANURIQUE PURS ET PROCEDE D'OBTENTION DE CES
POLYISOCYANATES

La présente invention concerne des polyisocyanates à groupement isocyanurique très purs.

Elle concerne plus spéfiquement les polyisocyanates à groupement isocyanurique préparés par cyclotrimérisation catalytique d'isocyanates aliphatiques, cycloaliphatiques ou arylaliphatiques.

Plus précisément encore l'invention consiste en des polyisocyanates à groupement isocyanurique, obtenus par cyclotrimérisation catalytique d'isocyanates aliphatiques, cycloaliphatiques ou arylaliphatiques, et contenant moins de 0,03 % en poids d'isocyanate monomère et moins de 1,0 % en poids de dimère, par rapport au poids du polyisocyanate à groupement isocyanurique.

On connait de nombreux procédés de préparation de polyisocyanates à groupement isocyanurique par cyclotrimérisation d'isocyanates, notamment de diisocyantes, aliphatiques, cycloaliphatiques ou arylaliphatiques.

On peut par exemple les obtenir par cyclotrimérisation partielle des groupements NCO de polyisocyanates simples ou de polyisocyanates adducts, à l'aide de catalyseurs variés tels que les amines tertiaires (brevet allemand no 951 168), les dérivés des métaux alcalins ou alcalino-terreux tels que les hydroxydes, carbonates, alcoolates (brevet français no 1 190 065), les hydroxydes d'ammonium quaternaire (brevet français no 1 204 697 et 1 566 256 ; demandes de brevet européen no 03 765 et 10 589), les phosphines (brevets français no 1 510 342 et 2 023 423), les catalyseurs à groupement éthylèneimine (brevets français no 1 401 513 et 2 230 642) et les bases de Mannich (brevets français no 2 290459 et 2 332 274).

On peut également réaliser cette cyclotrimérisation partielle des groupements NCO en présence de composés aminosilylés tels que monoaminosilanes, les diaminosilanes, les silylurées et les silazanes décrits dans la demande de brevet européen 57 653.

Le catalyseur de cyclotrimérisation partielle des groupements NCO doit, de manière générale, être désactivé lorsque l'on a atteint la teneur désirée en groupements isocyanate libres. Cette désactivation peut être effectuée par addition d'un composé acide (hydracide, chlorure d'acide...), d'un agent alkylant (iodure de méthyle par exemple), d'un agent acylant. Elle peut également être réalisée par un traitement thermique approprié.

En fin de réaction de cyclotrimérisation, le polyisocyanate à groupement isocyanurique est séparé par distillation de l'isocyanate n'ayant pas réagi et du solvant éventuel, lorsque la réaction est conduite en milieu solvant.

Pour cela, il est nécessaire de chauffer le mélange réactionnel final, par exemple dans un évaporateur, ce qui permet d'éliminer la majeure partie de l'isocyanate résiduel et du solvant éventuel. Ensuite il faut, le plus souvent, procéder à une nouvelle distillation plus poussée, afin de tenter de faire disparaitre les dernières traces d'isocyanate.

Cependant il s'avère que, même après ces différents traitements, il demeure toujours dans le polyisocyanate à groupement isocyanurique une quantité d'isocyanate au moins égale à 0,1 ou 0,2 % en poids par rapport au polyisocyanate. Généralement le polyisocyanate trimère comporte aussi une quantité de dimère d'environ 2 à 5 % en poids par rapport au polyisocyanate.

Il a été proposé dans la demande de brevet européen no 105 242 de réduire la teneur en isocyanate monomère par traitement du polyisocyanate à l'aide de 2 à 30 % d'un solvant inerte, dans un évaporateur en couche mince. Ce procédé comprend néanmoins une phase de chauffage du polyisocyanate vers 140°C à 150°C et implique l'addition d'un solvant qu'il sera ensuite nécessaire d'éliminer.

Un des buts de la présente invention concerne, comme cela a été indiqué précédemment, des polyisocyanates à groupement isocyanurique, préparés par cyclotrimérisation catalytique d'au moins un diisocyanate aliphatique, cycloaliphatique ou arylaliphatique dont les groupements isocyanate ne sont pas directement liés à un cycle aromatique, ayant une teneur en diisocyanate de départ inférieure ou égale à 0,03 % en poids, d'une en dimère inférieure ou égale à 1,0 % en poids, par rapport audit polyisocyanate.

L'invention concerne également de tels polyisocyanates à groupement isocyanurique ayant une teneur en diisocyanate de départ inférieure ou égale à 0,01 % en poids et une teneur en dimère inférieure ou égale à 0,5 %, par rapport audit polyisocyanate.

A titre d'exemples non limitatifs de ces diisocyanates monomères, on peut citer :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,

- le bis(isocyanato-4 cyclohexyl)méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

Les diisocyanates monomères peuvent être utilisés séparément ou sous forme de mélanges de plusieurs d'entre eux.

Ainsi, par exemple, le diisocyanato-1,6 hexane, qui est l'un des diisocyanates monomères préférés, peut être utilisé seul ou en mélanges, notamment avec le diisocyanato-1,5 méthyl-2 pentane et/ou le diisocyanato-1,4 éthyl-2 butane ; des mélanges de ces deux derniers diisocyanates peuvent également être utilisés.

Les polyisocyanates à groupement isocyanurique peuvent être utilisés notamment pour préparer des peintures et vernis polyuréthannes non-jaunissants. Les diisocyanates libres présentent une relative toxicité, il est particulièrement intéressent de disposer de polyisocyanates à groupement isocyanurique, à très faible taux de diisocyanate résiduel. D'autre part le dimère de l'isocyanate de départ, qui se forme en plus ou moins grande quantité pendant la réaction de cyclotrimérisation, est susceptible dans certains conditions de chauffage de libérer à nouveau en partie du diisocyanate libre. Ce risque est pratiquement éliminé lorsque l'on dispose de polyisocyanates à groupement isocyanurique ayant une faible teneur en dimère.

Un procédé simple et efficace d'obtention de polyisocyanates à groupement isocyanurate à faibles teneurs en diisocyanate de départ et en dimère de ce diisocyanate, consiste à extraire, après la réaction de cyclotrimérisation, l'excès de diisocyanate monomère et le dimère formé, à l'aide d'un gaz inerte à l'état liquide ou à l'état supercritique.

Comme gaz inertes, on peut citer à titre d'exemples le gaz carbonique, le butane, l'éthane, le propane ou l'éthylène.

Le gaz carbonique, qui est bon marché, non-toxique et ininflammable, est le composé utilisé le plus fréquemment, à l'état liquide ou supercritique.

L'extraction peut se faire en continu ou en discontinu.

A l'état liquide le gaz carbonique est généralement mis en oeuvre à une température comprise entre 0°C et 31°C et sous une pression de 30 à 500 bars.

Il est préférable d'opérer entre 20°C et 31°C, afin de ne pas avoir une viscosité trop importante du polyisocyanate à traiter, et sous une pression de 60 à 300 bars.

A l'état supercritique la température est supérieure à la températue critique du gaz carbonique (31,4°C) et la pression est généralement de 73 à 500 bars. De préférence la pression est de 73 à 350 bars et la température est comprise entre 31,4°C et 100°C.

Il est possible de traiter la masse réactionnelle dès la fin de la réaction de cyclotrimérisation.

Cependant, compte-tenu des quantités importantes de diisocyanate monomère en excès, il peut êtr préférable et plus économique de procéder préalablement à une élimination rapide de la plus grande partie du diisocyanate monomère en excès.

Ce traitement, qui est souvent une évaporation très rapide sous pression réduite, ne nécessite pas de chauffer à une température élevée.

En pratique donc, la masse obtenue après la réaction de cyclotrimérisation est envoyée dans un évaporateur à couche mince, qui permet de séparer environ 80 à 90 % du diisocyanate monomère en excès.

Le polyisocyanate à groupement isocyanurique, contenant une quantité encore relativement importante de diisocyanate monomère est alors traité à l'aide de $CO_2$ liquide ou de $CO_2$ à l'état supercritique.

On peut opérer en discontinu, par exemple en mélangeant dans un réacteur le polyisocyanate à groupement isocyanurique que l'on veut purifier avec le gaz carbonique liquide ou à l'état supercritique que l'on y a introduit.

Après séparation du gaz carbonique, contenant le diisocyanate monomère et le dimère, du polyisocyanate à groupement isocyanurique purifié, on peut séparer ledit gaz carbonique des extraits par détente et/ou augmentation de température.

L'extraction peut être contuide de manière conventionnelle, dans un appareillage connu en soi.

A partir d'une source de gaz carbonique, on envoie ledit gaz dans un échangeur thermique où il est liquéfié ; il est ensuite véhiculé, à la pression désirée, à l'aide d'une pompe, vers un autre échangeur thermique dans lequel il est porté à la température choisie pour l'extraction.

La gaz carbonique, à l'atat liquide ou à l'état supercritique, est ensuite envoyé dans l'appareil d'extraction, qui peut être par exemple une colonne remplie d'un garnissage permettant un meilleur contact entre le polyisocyanate à groupement isocyanurique et le gaz carbonique.

Le polyisocyanate à groupement isocyanurique peut être introduit à l'autre extrémité de la colonne : on a alors une extraction à contre-courant. Moins fréquemment, on peut l'introduire à la même extrémité que le gaz d'extraction : on a alors une extraction à co-courant.

Le polyisocyanate à groupement isocyanurique purifié est récupéré à une extrémité de la colonne d'extraction, tandis que le gaz carbonique, chargé du diisocyanate monomère, du dimère de ce diisocyanate et d'un peu de polyisocyanate, est soumis à un traitement pour le séparer des composés extraits.

Le gaz carbonique extrayant une certaine quantité de polyisocyanate à groupement isocyanurique, il est intéressant en pratique de récupérer l'essentiel de ce polyisocyanate. Pour cela on peut procéder à une séparation fractionnée des extraits consistant à modifier le pouvoir solvant du gaz carbonique contenant les extraits. Le polyisocyanate à groupement isocyanurique qui est moins soluble que le diisocyanate et que le dimère peut ainsi être séparé. Cette modification du pouvoir solvant du gaz carbonique peut se faire par diminution de sa pression ou le plus souvent par augmentation de sa température.

Le gaz carbonique chargé essentiellement du diisocyanate et du dimère est ensuite soumis à un traitement afin de le séparer de ces composés extraits.

Comme précédemment pour le polyisocyanate à groupement isocyanurique, on peut pour cela agir soit par diminution de sa pression, soit par augmentation de sa température.

La diminution de pression, ou détente, peut se faire en une ou plusieurs étapes et le gaz carbonique peut être détendu jusqu'à une pression égale à la pression atmosphérique ou jusqu'à une pression plus élevée, sous laquelle il sera recyclé dans le cas d'un procédé continu.

En effet, si le gaz carbonique est recyclé, il est économiquement préférable de ne pas le détendre jusqu'à la pression atmosphérique, ce qui nécessiterait une plus grande dépense d'énergie pour le recomprimer dans le cycle suivant du procédé. Il est préférable de ne le détendre que jusqu'à une pression sous laquelle les composés extraits ne sont pas, ou très peu, solubles.

On peut également séparer le gaz carbonique de la totalité des extraits qu'il contient comme indiqué précédemment et faire subir à ce mélange d'extraits, comprenant le diisocyanate monomère, le dimère et la partie de trimère à groupement isocyanurique extraite, une nouvelle extraction par le gaz carbonique à l'état liquide ou supercritique ; cette seconde extraction, effectuée sur un mélange beaucoup moins riche en polyisocyanate à groupement isocyanurate que le mélange issu du procédé de cyclotrimérisation, entraine beaucoup moins de trimère.

On peut également recycler la totalité des extraits, comprenant le diisocyanate monomère, le dimère et la partie de trimère extraite, dans la phase de préparation du trimère à groupement isocyanurique. La concentration du produit en dimère augmentera au fur et à mesure des recyclages ; ou pourra alors, après un certain nombre de recyclages, procéder, selon l'une des variantes précédentes, pour séparer le dimère et le diisocyanate monomère de la partie extraite du trimère à groupement isocyanurique.

Comme cala a été indiqué précédemment, le procédé peut être mis en oeuvre de manière continue ou discontinue et l'appareillage utilisé n'est pas limité à la description du principe de fonctionnement décrit ci-avant.

Généralement, dans le cadre du procédé de l'invention, on préfère mettre en oeuvre le gaz carbonique à l'état supercritique.

Les exemples qui suivent illustrent la présente invention.

EXEMPLE 1

Dans une colonne en acier inoxydable, de 19 mm de diamètre et de 250 mm de hauteur, remplie de garnissage Dixon, on introduit 90,8 g de trimère à groupement isocyanurique préparé à partie de diisocyanato-1,6 hexane (HDI). La teneur de ce polyisocyanate est de 4,3 % en poids d'HDI libre et d'environ 2,5 % en poids de dimère.

La colonne est thermostatée à 20°C. On admet le gaz carbonique à l'état liquide jusqu'à une pression de 100 bars.

La pression de détente de la colonne d'extraction est maintenue à la valeur désirée par un régulateur de pression.

Lorsque le système est en équilibre, on fait passer par le bas de la colonne le gaz carbonique à l'état liquide, avec un débit de 800 g heure environ.

Les extraits sont récupérés, au delé du régulateur de pression par détente à pression atmosphérique, dans une succession de flacons.

La teneur en HDI du polyisocyanate traité est contrôlée par chromatographie en phase liquide.

Le tableau (I) ci-après rassemble les résultats de l'essai :
- quantité d'extrait (HDI + dimère + trimère)
- rapport pondéral extrait/polyisocyanate chargé
- teneur en HDI du polyisocyanate traité, en fonction de la quantité de $CO_2$ liquide engagée
- teneur en dimère du polyisocyanate traité, en fonction de la quantité de $CO_2$ liquide engagée.

| Poids cumulé de CO2 liquide utilisé | Poids cumulé d'extrait | Extrait/poly-isocyanate chargé % en poids | Teneur en HDI % en poids | Teneur en dimère % en poids |
|---|---|---|---|---|
| 500 g | 5,4 g | 6,0 | 0,32 | 1,2 |
| 1000 g | 7,8 g | 8,6 | 0,06 | 1,1 |
| 1500 g | 9,45 g | 10,4 | 0,008 | 0,7 |
| 2500 g | 10,8 g | 11,9 | 0,005 | 0,5 |

## Tableau (I)

### EXEMPLE 2

Dans un autoclave de 45 cm3, comportant deux regards en verre, on introduit 20,1 g de trimère à groupement isocyanurique préparé à partir de l'HDI. La teneur de ce polyisocyanate est de 0,18 % d'HDI libre et d'environ 2,5 % en poids de dimère.

L'autoclave est thermostaté à 35°C. On admet le gaz carbonique à l'état supercritique jusqu'à une pression de 100 bars. La pression de détente de l'autoclave est maintenue à la valeur désirée par un régulateur de pression.

Lorsque le système est en équilibre, on fait passer par le bas de l'autoclave, le gaz carbonique à l'état supercritique, avec un débit de 700 g/heure environ.

Les extraits sont récupérés dans les conditions décrites pour l'exemple 1.

Les résultats obtenus sont indiqués ci-après :
- poids de $CO_2$ supercritique utilisé : 5000 g
- poids d'extrait : 2,2 g
- extrait/polyisocyanate chargé (% en poids) : 10,95 %
- teneur en HDI dans le polyisocyanate traité (% en poids) : 0,005 %
- teneur en dimère dans le polyisocyanate traité (% en poids) : 0,4 %.

### EXEMPLE 3

Réalisation d'un essai en marche continue.

L'appareillage utilisé est constitué :
- d'une colonne d'extraction en acier inoxydable ayant un diamètre intérieur de 16 mm et une longueur de 1 mètre remplie d'anneaux DIXON en acier inodydable de 3 mm x 3mm ; cette colonne comporte une double enveloppe pour circulation du fluide de chauffage ;
- d'une pompe d'alimentation du trimère de l'HDI à traiter en haut de la colonne ;
- d'une pompe d'alimentation de gaz carbonique en bas de la colonne ;
- d'une colonne d'extraction du trimère purifié.

On injecte en haut de la colonne thermostatée à 40°C, le trimère de l'HDI contenant 1,1 % en poids de l'HDI libre. La trimère est préchauffé avant la pompe d'alimentation.

Il est injecté avec un débit de 130 grammes/heure tandis que le $CO_2$ supercritique est injecté au bas de la colonne (40°C, 200 bars) avec un débit de 2600 g/heure.

L'essai est poursuivi pendant 5 heures en continu.

On soutire en continu le trimère de l'HDI purifié à raison de 110 g/heure.

Le trimère purifié contient 0,003 % de l'HDI libre.

## Revendications

1° - Polyisocyanate à groupement isocyanurique préparé par cyclotrimérisation catalytique d'au moins un diisocyanate aliphatique, cycloaliphatique ou arylaliphatique, caractérisé en ce qu'il contient en poids par rapport audit polyisocyanate :
- une quantité de diisocyanate libre de départ égale ou inférieure à 0,03 %,
- une quantité de dimère du diisocyanate de départ égale ou inférieure à 1,0%.

2° - Polyisocyanate à groupement isocyanurique selon la revendication 1, caractérisé en ce qu'il est préparé à partir d'au moins un des diisocyanates monomères suivants :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,
- le bis(isocyanato-4 cyclohexyl)méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

3° - Polyisocyanate à groupement isocyanurique selon l'une des revendications 1 ou 2, caractérisé en ce qu'il est préparé à partir de diisocyanato-1,6 hexane, seul ou en mélange avec le diisocyanato-1,5 méthyl-2 pentane et/ou le diisocyanato-1,4 éthyl-2 butane.

4° - Polyisocyanate à groupement isocyanurique selon l'une des revendications 1 à 3, caractérisé en ce qu'il contient en poids par rapport audit polyisocyanate :
- une quantité de diisocyanate libre de départ égale ou inférieure à 0,01 %,
- une quantité de dimère du diisocyanate de départ égale ou inférieure à 0,5 %.

5° - Procédé d'obtention d'un polyisocyanate à groupement isocyanurique selon l'une des revendications 1 à 4, caractérisé en ce que le polyisocyanate à groupement isocyanurique brut, préparé par cyclotrimérisation catalytique, est traité par un gaz inerte à l'état liquide ou supercritique.

6° - Procédé selon la revendication 5, caractérisé en ce que le gaz inerte utilisé à l'état liquide ou supercritique est le gaz carbonique.

7° - Procédé selon la revendication 6, caractérisé en ce que l'on opère à une température comprise entre 0 et 31°C et sous une presion de 30 à 500 bars.

8° - Procédé selon l'une des revendications 6 et 7, caractérisé en ce que l'on opère à une température comprise entre 20 et 31°C et sous une pression de 60 à 300 bars.

9° - Procédé selon la revendication 6, caractérisé en ce que l'on opère à une température supérieure à la température critique du gaz carbonique, de préférence comprise entre 31,4°C et 100°C et sous une pression de 73 à 500 bars et de préférence de 73 à 350 bars.

10° - Procédé selon l'une des revendications 5 à 9, caractérisé en ce que l'on élimine, préalablement à l'extraction par le gaz inerte à l'état liquide ou supercritique, la plus grande partie de l'excès de diisocyanate monomère.

11° - Procédé selon l'une des revendications 6 à 10, caractérisé en ce que l'on récupère le polyisocyanate à groupement isocyanurique dissout dans le gaz carbonique avant de séparer le gaz carbonique d'une part et le diisocyanate et le dimère d'autre part, par diminution de la pression et/ou augmentation de la température du gaz carbonique.